# EUROPÄISCHE PATENTSCHRIFT

(11) **EP 4 333 748 B1**
(45) Veröffentlichungstag und Bekanntmachung des Hinweises auf die Patenterteilung: **04.02.2026**
(21) Anmeldenummer: 22725799.5
(22) Anmeldetag: 26.04.2022
(51) Int. Cl.: A61B 17/80

(54) **MEDIZINISCHES IMPLANTAT ZUR OSTEOSYNTHESE**
MEDICAL IMPLANT FOR OSTEOSYNTHESIS
IMPLANT MÉDICAL POUR OSTÉOSYNTHÈSE

(30) Priorität: 05.05.2021 DE 102021111653
(43) Veröffentlichungstag der Anmeldung: 13.03.2024
(73) Patentinhaber: Rheinisch-Westfälische Technische Hochschule Aachen, Abgekürzt RWTH Aachen, Körperschaft Des Öffentlichen Rechts, 52062 Aachen (DE)
(72) Erfinder: ALABDULRAHMAN, Hatem, 52146 Würselen (DE); GREVEN, Johannes, 41061 Mönchengladbach (DE); HORST, Klemens, 52072 Aachen (DE); ESCHWEILER, Jörg, 52396 Heimbach-Vlatten (DE)
(74) Vertreter: Eisenführ Speiser
(86) Internationale Anmeldenummer: PCT/EP2022/061066
(87) Internationale Veröffentlichungsnummer: WO 2022/233643

(56) Entgegenhaltungen:
- CN-Y- 2 850 538
- DE-B1- 2 603 087
- RU-U1- 14 498
- US-A1- 2019 008 569
- US-A1- 2019 183 549

## Beschreibung

Die Erfindung betrifft ein medizinisches Implantat zur Osteosynthese von Symphysenrupturen am menschlichen Becken, aufweisend eine kraniale Platte, die dazu ausgebildet ist, von einer kranialen Seite an einem ersten Schambein und einem zweiten Schambein eines zu behandelnden Patienten befestigt zu werden, um die Schambeine zu verbinden. Ferner betrifft die Erfindung ein Verfahren zur Osteosynthese einer Symphysenruptur an einem Trainingskörper zu Trainingszwecken.

In den letzten Jahrzehnten ist die Weiterentwicklung verschiedener Operationstechniken und die Behandlung von Knochenbrüchen mit Hilfe von Implantaten, wie z.B. Marknägeln oder Osteosyntheseverfahren mittels Platten, stetig vorangeschritten. Dennoch gibt es Teilbereiche, in denen die weitere Entwicklung stagniert.

Das Verletzungsbild des polytraumatisierten Patienten und insbesondere Frakturen des Beckenrings stellen die unfallchirurgische Versorgung auch heutzutage noch vor große Herausforderungen, auch wenn sich die Versorgung in den letzten Jahrzehnten stetig verbessert hat, so sind viele Versorgungstrategien bis heute nicht ausreichend validiert oder stagnieren in Bezug auf ihre Weiterentwicklung. Aufgrund ihrer hohen Letalität sind Beckenringfrakturen in Bezug auf die Stabilisierung und Versorgung von hoher Bedeutung.

Oftmals wird zur Osteosynthese, das heißt zur operativen Verbindung zweier Knochen, von Beckenringfrakturen ein einfaches Plattenimplantat verwendet, das kranial, also Kopfseitig, auf dem Beckenkamm implantiert wird. Auch Rupturen der Symphyse (Schambeinfuge) des menschlichen Beckens werden heute durch einfache Plattenimplantate versorgt, wobei das Plattenimplantat von einer kranialen Seite an einem ersten Schambein und einem zweiten Schambein eines zu behandelnden Patienten befestigt wird und so die beiden Hemisphären des Beckens verbindet. Aufgrund der hohen physiologischen Belastung kommt es bei solchen einfachen Plattenimplantaten häufig zum Implantatversagen, beispielsweise zu Verformungen oder zum Lösen des Implantats.

Ferner ist es auch bekannt, Implantate zur Behandlung von Beckenringfrakturen zu verwenden, die komplexer gestaltet sind als einfache Platten. RU 2 739 692 offenbart ein Plattensystem zur Rekonstruktion alter Beckenringfrakturen bei männlichen Patienten. Das offenbarte Implantat weist eine kraniale Platte auf, die mit einer u-förmigen zweiten Platte verbunden ist. Die zweite Platte ist dazu vorgesehen, von außen an die zu verbindenden Beckenknochen angelegt zu werden. Nachteilig an dem offenbarten Implantat ist, dass dieses nur für die Behandlung männlicher Patienten vorgesehen ist, und dass die zweite Platte von außen am Becken befestigt werden muss. Bei der Behandlung von Symphysenrupturen kann es aufgrund der an einer Außenseite des Beckens ansetzenden Bauchmuskulatur zu erheblichen Beeinträchtigungen des Patienten kommen. Ferner behindert die von der Außenseite anliegende Platte die Sicht eines Operateurs auf die Symphyse, wodurch das Risiko für eine fehlerhafte Positionierung des Implantats steigt. Die Osteosynthese kann mit dem vorgeschlagenen Implantat nur von sehr erfahrenen Ärzten durchgeführt werden.

Ferner sind aus CN 205126393 U und CN 204995566 U Plattensysteme zur Behandlung von Beckenringfrakturen bekannt, die aber nicht zur Behandlung von Symphysenrupturen geeignet sind. Das in CN 205126393 U offenbarte Implantat ist zur Osteosynthese von Frakturen der Beckenpfanne (Acetabulum) vorgesehen, während die in CN 204995566 U offenbarte Platte zur Osteosynthese von Frakturen des Iliosakralgelenks vorgesehen ist. Typische Problemstellungen der Osteosynthese von Symphysenrupturen, wie beispielsweise das korrekte Einstellen eines Symphsenabstands zwischen den Schambeinen, müssen bei Frakturen des Acetabulum und des Iliosakralgelenks nicht beachtet werden, da die zu verbindenden Knochen einfach aneinander angelegt werden können. Die Implantation ist durch eine optische Behinderung des Operateurs kaum bzw. nur wenig erschwert.

Von der Firma Stryker ist das "PRO Pelvis and Acetabulum System" bekannt. Zur Behandlung von Brüchen des Acetabulum umfasst das System "PRO quadilateral surface plates", die von innerhalb des Beckenrings an das Acetabulum angesetzt werden. Im Gegensatz zur Symphyse, für die das PRO Pelvis and Acetabulum System nur einfache Platten offenbart, ist die Beckenpfanne im Rahmen einer operativen Behandlung erheblich leichter zugänglich und einzusehen, insbesondere da der Zugang in der Regel vom Bauch des Patienten erfolgt und so eine schräge Sicht auf die Beckenpfanne erlaubt.

RU 14 498 U1 offenbart ein Implantat zur Osteosynthese von Symphysenrupturen mit zwei plattenartigen Elementen, die über rechtwinklige Abschnitte miteinander verbunden sind.

Es ist Aufgabe der vorliegenden Erfindung, ein medizinisches Implantat zur Osteosynthese von Symphysenrupturen am menschlichen Becken anzugeben, das gegenüber dem Stand der Technik eine verbesserte Zuverlässigkeit gegenüber Implantatversagen aufweist und Patientenschonender eingesetzt werden kann, sowie eine vereinfachte Implantation ermöglicht.

Die Erfindung löst die Aufgabe bei einem medizinischen Implantat der eingangs genannten Art durch eine posteriore Platte, die dazu ausgebildet ist, von einer posterioren Seite an dem ersten Schambein und dem zweiten Schambein des Patienten befestigt zu werden, um die Schambeine zu verbinden, wobei die kraniale Platte und die posteriore Platte durch wenigstens einen ersten Verbindungsarm miteinander verbunden sind. Die posteriore Seite ist die zu einer Innenseite des Beckenrings weisende Seite der Schambeine. Die posteriore Seite der Schambeine ist im Bereich der Symphyse dem Kreuzbein (Sakrum) des Patienten zugewandt. Die kraniale Seite ist eine dem Kopf des Patienten zugewandte Seite. Das erfindungsgemäße Implantat kann demnach im Rahmen der Osteosynthese einer Symphysenruptur von innen und oben an die Schambeine des zu behandelnden Patienten angelegt werden. Die Erfinder haben herausgefunden, dass es möglich ist, eine posteriore Platte von einer posterioren Seite der Schambeine anzulegen. Eine Beeinträchtigung der von außen an die Schambeine angreifenden Bauchmuskulatur des Patienten kann hierdurch verhindert werden. Ferner werden häufige Probleme des Implantatversagens, wie beispielsweise der Bruch eines einfachen Plattenimplantats, überwunden. Dies wird über eine gleichmäßigere Verteilung der Kräfte durch die mittels des Verbindungsarms verbundenen Platten erreicht. So können auftretende Kräfte und Drehmoment optimal auf die verwendeten Schrauben übertragen werden und ein Lockern des Implantats wird verhindert. Des Weiteren wird durch den Verbindungsarm eine optimale Ausrichtung der posterioren Platte und der kranialen Platte zueinander erreicht. Das medizinische Implantat kann daher besonders einfach und sicher angewendet werden, wodurch eine schonendere Behandlung des Patienten ermöglicht wird. Vorzugsweise sind die posteriore Platte und/oder die kraniale Platte dazu ausgebildet, im Wesentlichen vollflächig (über die gesamte Fläche der Platten) an den Schambeinen des Patienten anzuliegen. Es soll verstanden werden, dass die Platten auch dann vollflächig anliegen, wenn die Symphyse überbrückt wird. Vorzugsweise sind die posteriore Platte und/oder die kraniale Platte an eine generelle Geometrie der menschlichen Schambeine angepasst. Im Rahmen der Osteosynthese erlaubt eine von der posterioren Seite angelegte Platte eine ungehinderte Sicht des Operateurs auf die Symphyse. Das Implantieren des erfindungsgemäßes Implantat ist erheblich vereinfacht.

Der Verbindungsarm verbindet die kraniale Platte und die posteriore Platte. Bevorzugt ist ein Querschnitt des Verbindungsarms von einem Querschnitt der posterioren Platte und/oder der kranialen Platte verschieden, insbesondere kleiner. Vorzugsweise ist das medizinische Implantat in einer Sagittalebene L-förmig ausgebildet. Weiter bevorzugt ist der Verbindungsarm vollständig oder teilweise flexibel. Es kann aber auch vorgesehen sein, dass der Verbindungsarm vollständig oder teilweise steif ausgebildet ist. Ein Verbindungsarm ist steif, wenn er bei üblicher Handhabung im Rahmen einer Osteosynthese nicht Wesentlich verformt wird. Ein flexibler Verbindungsarm ist reversibel, insbesondere bereits durch das Eigengewicht des Implantats hervorgerufene Kräfte, verformbar. Der Verbindungsarm kann vorzugsweise bogenförmig, insbesondere kreisbogenförmig, und/oder (mehr-)eckig ausgebildet sein. Das medizinische Implantat ist sowohl für teilweise als auch für vollständige Symphysenrupturen einsetzbar.

Gemäß einer ersten bevorzugten Ausführungsform ist der erste Verbindungsarm ein bezogen auf die posteriore Platte und die kraniale Platte außermittiger Verbindungsarm, er ist also vorzugsweise entlang der längsten Ausdehnung der kranialen Platte und/oder posterioren Platte außermittig angeordnet. Zur optimalen Kraftverteilung werden Implantate im Rahmen der Osteosynthese in der Regel symmetrisch zur Symphyse ausgerichtet. Durch einen außermittigen Verbindungsarm wird eine optimale Zugänglichkeit der Symphyse gewährleistet bzw. die Zugänglichkeit nicht behindert, da der Verbindungsarm bei weitgehend symmetrischer Ausrichtung des Implantats zur Symphyse versetzt angeordnet ist. Komplikationen können verhindert werden.

Vorzugsweise weist das medizinische Implantat einen zweiten Verbindungsarm auf, wobei der zweite Verbindungsarm, besonders bevorzugt ein bezogen auf die posteriore Platte und die kraniale Platte außermittiger Verbindungsarm ist. Der zweite Verbindungsarm kann vorzugsweise im Wesentlichen identisch zum ersten Verbindungsarm ausgebildet sein. Es kann jedoch beispielsweise und bevorzugt auch vorgesehen sein, dass der zweite Verbindungsarm einen zum ersten Verbindungsarm verschiedenen Querschnitt aufweist. Vorzugsweise sind der erste Verbindungsarm und/oder der zweite Verbindungsarm im Wesentlichen rechtwinklig mit der posterioren Platte und/oder der kranialen Platte verbunden. Rechtwinklig mit den Platten verbundene Verbindungsarme erstrecken sich dann im Wesentlichen quer zu einer Beckenringrichtung. Die Beckenringrichtung wird durch die Form des menschlichen Beckens in einer Transversalebene definiert, d.h. bei Betrachtung in superiorer-inferiorer Richtung. Näherungsweise entspricht die Beckenringrichtung der Umfangsrichtung eines Kreises oder einer Ellipse. Der erste Verbindungsarm und/oder der zweite Verbindungsarm kann sich jedoch auch sowohl quer als auch entlang der Beckenringrichtung, beispielsweise schräg zur Beckenringrichtung, erstrecken.

Erfindungsgemäß weist das medizinische Implantat ferner einen Positioniervorsprung zum zumindest teilweisen Eingreifen in die Symphyse des Beckens des zu behandelnden Patienten auf. Der Positioniervorsprung kann im Rahmen der Osteosynthese in die Symphyse eingreifen und so in Beckenringrichtung an eine der Symphyse zugewandte Seite eines Schambeins angelegt werden. Der Positioniervorsprung erleichtert so wesentlich das korrekte Ausrichten des medizinischen Implantats an einem ersten Schambein. So kann das Implantat in korrekter Ausrichtung an einem ersten Schambein befestigt werden, bevor die beiden Schambeine bzw. die beiden Hüft-Hemisphären des Patienten zueinander ausgerichtet werden. Die zweite Hemisphäre kann dann genau entlang der Platte geführt und befestigt werden. Durch die beiden Platten sind die Hemisphären horizontal und vertikal miteinander verbindbar. Mittels des erfindungsgemäßen Implantats ist es nicht mehr notwendig die korrekte Ausrichtung der Schambeine vorab sicherzustellen, bevor die Platten an den beiden Schambeinen befestigt werden. Ausrichtungsfehler werden effektiv verhindert und Patienten geschont. Vorzugsweise kann auch der erste Verbindungsarm als Positioniervorsprung zum zumindest teilweisen Eingreifen in die Symphyse ausgebildet sein. Das medizinische Implantat kann auch einen zweiten Positioniervorsprung oder mehr als zwei Positioniervorsprünge aufweisen.

Bevorzugt ist der Positioniervorsprung zwischen dem ersten Verbindungsarm und dem zweiten Verbindungsarm angeordnet. Vorzugsweise ist dann jedem Schambein des Patienten zumindest ein Verbindungsarm zugeordnet, wodurch die Stabilität des Implantats verbessert wird. Ferner wird das Einsetzen des Positioniervorsprungs in die Symphyse des Patienten nicht durch die Verbindungsarme behindert. Vorzugsweise ist der Positioniervorsprung mittig zwischen den Verbindungsarmen angeordnet, weist also zum ersten Verbindungsarm und zum zweiten Verbindungsarm einen im Wesentlichen gleichen Abstand auf. Der Verbindungsarm ist bevorzugt einstückig mit der posterioren Platte und/oder der kranialen Platte ausgebildet.

In einer bevorzugten Ausführungsform ist der Positioniervorsprung mittig am Implantat, vorzugsweise in einer Symmetrieebene des Implantats, angeordnet. Die mittige Anordnung des Positioniervorsprungs wird in Beckenringrichtung betrachtet. Der Positioniervorsprung ist vorzugsweise mittig zwischen einem ersten Ende des medizinischen Implantats, das beim Positionieren des Positioniervorsprungs in der Symphyse in Beckenringrichtung am weitesten von der Symphyse entfernt ist, und einem gegenüberliegenden zweiten Ende des medizinischen Implantats, das beim Positionieren des Positioniervorsprungs in der Symphyse in Beckenringrichtung am weitesten von der Symphyse entfernt ist, angeordnet. Vorzugsweise ist der Positioniervorsprung in einer Symmetrieebene des Implantats, die senkrecht zur Beckenringrichtung ist, angeordnet.

Bevorzugt weist der Positioniervorsprung eine stumpfe Stirnseite auf. Die Stirnseite ist vorzugsweise abgerundet ausgebildet und weist besonders bevorzugt keine Kanten auf. Eine stumpfe Stirnseite verhindert Verletzungen der Symphyse und/oder der der Symphyse zugewandten Seiten der Schambeine. Vorzugsweise ist die Stirnseite des Positioniervorsprungs kuppelförmig ausgebildet. Es kann aber auch vorgesehen sein, dass die Stirnseite flach ist und abgerundete Übergänge zu Seitenwänden des Vorsprungs aufweist. In einer bevorzugten Weiterbildung ist der Positioniervorsprung ein quer zu einer Vorsprungshöhe des Vorsprungs langgestreckter Steg. Ein langgestreckter Steg bietet eine besonders große Anlagefläche und erlaubt so ein sicheres Positionieren. Die Vorsprungshöhe wird in der Richtung ermittelt, in der der Positioniervorsprung bei bestimmungsgemäßem Gebrauch des Implantats in die Symphyse eingreift. Erstreckt sich der Positioniervorsprung von der kranialen Platte, dann wird die Vorsprungshöhe in superior-inferiorer Richtung bestimmt. Wenn sich der Positioniervorsprung hingegen von der posterioren Platte erstreckt, dann wird die Vorsprungshöhe in posterior-anteriorer Richtung ermittelt.

In einer bevorzugten Ausgestaltung erstreckt sich der der Positioniervorsprung im Wesentlichen senkrecht von einer Anlagefläche an der kranialen Platte und/oder der posterioren Platte des medizinischen Implantats. Ein Verrutschen des Positioniervorsprungs kann so effektiv verhindert werden. Es kann aber auch vorgesehen sein, dass der Positioniervorsprung sich winklig von der Anlagefläche erstreckt. Beispielsweise kann der Positioniervorsprung keilförmig ausgebildet sein. Ein keilförmiger Positioniervorsprung erleichtert das Einsetzen in die Symphyse.

Vorzugsweise ist der Positioniervorsprung an der posterioren Platte vorgesehen. Durch das Anordnen des Positioniervorsprungs an der posterioren Platte kann ein korrekter Sitz des Implantats im Rahmen der Osteosynthese gut überprüft werden. Das Risiko einer falschen Positionierung und/oder für Komplikationen wird reduziert. Der Positioniervorsprung kann aber auch an der kranialen Platte vorgesehen sein. Vorzugsweise erstreckt sich der Positioniervorsprung quer zur Vorsprungshöhe vollständig von einer inferioren Seite der posterioren Platte bis zu einer superioren Seite der posterioren Platte. Hierdurch wird eine besonders große Anlagefläche erreicht.

Bevorzugt weist der Positioniervorsprung eine Vorsprungshöhe in einem Bereich von 0,5 mm bis 20 mm, bevorzugt 0,5 mm bis 15 mm, bevorzugt 0,5 mm bis 12 mm, bevorzugt 1 mm bis 12 mm, bevorzugt 1 mm bis 10 mm, bevorzugt 1 mm bis 8 mm, bevorzugt 2 mm bis 8 mm, bevorzugt 2 mm bis 6 mm, bevorzugt 2 mm bis 5 mm, besonders bevorzugt 3 mm bis 5 mm, auf. Die Randwerte der Bereiche sind ebenfalls bevorzugt. Eine Vorsprungshöhe im beanspruchten Bereich ermöglicht ein sicheres Anlegen und behindert zeitgleich nicht den Heilungsprozess der Symphyse.

In einer weiteren bevorzugten Ausgestaltung weist die posteriore Platte erste Befestigungslöcher auf, die zum Aufnehmen von ersten Verbindungsmitteln vorgesehen sind, wobei die kraniale Platte zweite Befestigungslöcher aufweist, die zum Aufnehmen von zweiten Verbindungsmitteln vorgesehen sind, und wobei die ersten Befestigungslöcher oder die zweiten Befestigungslöcher als Ovallöcher ausgebildet sind. Vorzugsweise sind die ersten Befestigungslöcher als Ovallöcher ausgebildet. Ovallöcher weisen in einer ersten Richtung quer zu einer Durchgangsrichtung, in der sich Verbindungsmittel durch die Löcher erstrecken, eine größere Erstreckung auf als in einer zweiten Richtung. Ovallöcher haben einen von einer Kreisform verschiedenen Querschnitt. So können Ovallöcher beispielsweise oval oder elliptisch ausgebildet sein. Vorzugsweise können Ovallöcher aber auch Langlöcher mit parallelen Seiten oder quer zur Durchgangsrichtung rechteckig ausgebildet sein. Es soll verstanden werden, dass Ovallöcher nicht auf einen ovalen Querschnitt beschränkt sind. Ovallöcher erlauben ein Bohren mit zur Durchgangsrichtung des Ovallochs geneigter Hauptachse des Bohrers. So müssen im Rahmen der Osteosynthese oftmals Befestigungslöcher in einen Knochen des zu behandelnden Patienten gebohrt werden, in den die Befestigungsmittel verankert werden. Die Zugänglichkeit im Rahmen einer Operation ist eingeschränkt. Ovallöcher ermöglichen, dass ein zum Bohren verwendeter Bohrer nicht senkrecht zum Knochen gehalten werden muss und vereinfachen so eine medizinische Versorgung des Patienten. Verletzungen des umgebenden Gewebes werden minimiert, Fehler verhindert und der Patient geschont. Es kann alternativ auch vorgesehen sein, dass sowohl die ersten Befestigungslöcher als auch die zweiten Befestigungslöcher als Ovallöcher ausgebildet sind.

Vorzugsweise ist eine Längsachse der Ovallöcher im Wesentlichen entlang einer Beckenringrichtung orientiert. Die Längsachse kennzeichnet die größte lichte Weite der Ovallöcher quer zur Durchgangsrichtung. Der Erfinder hat herausgefunden, dass durch eine Orientierung der Längsachse der Ovallöcher in Beckenringrichtung das Bohren weiter vereinfacht werden kann.

In einer bevorzugten Weiterbildung weist der Verbindungsarm eine geringere Steifigkeit auf als die posteriore Platte und die kraniale Platte, sodass eine Ausrichtung der posterioren Platte relativ zur kranialen Platte durch plastisches Verformen des Verbindungsarms einstellbar ist. Wenn das medizinische Implantat mehrere Verbindungsarme aufweist, dann ist eine Gesamtsteifigkeit der Verbindungsarme bevorzugt geringer als die Steifigkeit der posterioren Platte und der kranialen Platte. Durch die geringere Steifigkeit kann der Verbindungsarm verformt werden, während die posteriore Platte und die kraniale Platte sich nicht bzw. nicht wesentlich Verformen. Durch plastisches Verformen des Verbindungsarms ist das medizinische Implantat an die spezifischen Gegebenheiten des zu behandelnden Patienten anpassbar. So kann vorzugsweise ein von den Platten eingeschlossener Winkel variiert werden. Die relevante Steifigkeit ist vorzugsweise eine Steifigkeit gegenüber Verformung in einer Ebene quer zur Beckenringrichtung (bspw. einer durch die Symphyse verlaufende Sagittalebene). Bevorzugt weist der Verbindungsarm eine Länge von etwa 25 mm und vorzugsweise eine Breite von etwa 10 mm auf.

Vorzugsweise ist die kraniale Platte in einer Transversalebene konvex ausgebildet und/oder die posteriore Platte in der Transversalebene konvex ausgebildet ist. Eine konvexe Platte ist im Rahmen der Erfindung zum Bauch des Patienten bzw. nach außen gekrümmt. Eine konvexe posteriore Platte ist besonders gut an die natürliche Gestalt des Beckenrings angepasst. Durch eine konvexe kraniale Platte wird eine besonders große Anlagefläche erreicht. Konvexe Platten verhindern ferner ein Hervorstehen des Implantats nach dem Implantieren und verhindern so Verletzungen des umliegenden Gewebes.

In einer Ausführungsform sind ein erster Implantatabschnitt zur Befestigung an dem ersten Schambein und ein zweiter Implantatabschnitt zur Befestigung an dem zweiten Schambein im Wesentlichen spiegelsymmetrisch zueinander ausgebildet. Der erste Implantatabschnitt umfasst diejenigen Abschnitte der posterioren Platte und der kranialen Platte, die zum Befestigen an dem ersten Schambein vorgesehen sind und der zweite Implantatabschnitt umfasst analog die Abschnitte der posterioren Platte und der kranialen Platte die zum Befestigen an dem zweiten Schambein vorgesehen sind. Eine spiegelsymmetrische Ausgestaltung erlaubt eine symmetrische Kraftverteilung und verhindert so ein Lockern und/oder Beschädigungen des Implantats. Es soll verstanden werden, dass zwischen dem ersten und dem zweiten Implantatabschnitt liegende Implantatabschnitte nicht spiegelsymmetrisch ausgebildet sein müssen, dies aber sein können.

Vorzugsweise ist die kraniale Platte, die posteriore Platte und/oder der Verbindungsarm oder das gesamte Implantat aus Stahl, Titan, einem pulverbeschichteten Metall und/oder einem medizinischen Kunststoff, vorzugsweise PEEK, gebildet. Vorzugsweise kann die kraniale Platte, die posteriore Platte, der Verbindungsarm und/oder der Positioniervorsprung auch Oberflächenstrukturierungen aufweisen. Besonders bevorzugt ist das medizinische Implantat mittels 3D-Druck (additiver Fertigung) hergestellt.

Bevorzugt ist eine Länge der posterioren Platte und eine Länge der kranialen Platte im Wesentlichen identisch. Die Länge der Platten wird in Beckenringrichtung ermittelt. Durch eine Ausgestaltung mit gleicher Länge der Platten kann eine optimale Einleitung auftretender Kräfte erreicht und ein Implantatversagen verhindert werden.

In einer bevorzugten Ausführungsform ist der zumindest eine Verbindungsarm in einer Beckenringrichtung zu Enden der posterioren Platte und/oder Ende der kranialen Platte beabstandet angeordnet. Der Verbindungsarm ist also nicht an den in Beckenringrichtung der Platten liegenden Enden der posterioren Platte und/oder der kranialen Platte angeordnet. Wenn das Implantat mehrere Verbindungsarme aufweist, sind vorzugsweise alle Verbindungsarme zu den Enden der Platten beabstandet ausgebildet.

Weiterhin löst die Erfindung die eingangs genannte Aufgabe durch ein Verfahren zur Osteosynthese einer künstlichen Symphysenruptur an einem Trainingskörper zu Trainingszwecken mittels eines medizinischen Implantats gemäß der vorstehend beschriebenen Erfindung, aufweisend die Schritte: Anlegen einer kranialen Platte des medizinischen Implantats an einem ersten Schambein des Trainingskörpers von einer kranialen Seite; Anlegen einer posterioren Platte des medizinischen Implantats an einem ersten Schambein des Trainingskörpers von einer posterioren Seite; Befestigen der kranialen Platte an dem ersten Schambein; Befestigen der posterioren Platte an dem ersten Schambein; Einstellen eines Symphysenabstands zwischen dem ersten Schambein und dem zweiten Schambein; Befestigen der kranialen Platte an dem zweiten Schambein und Befestigen der posterioren Platte an dem zweiten Schambein nach dem Einstellen des Symphysenabstands. Der Trainingskörper ist kein lebender menschlicher oder tierischer Körper. Das erfindungsgemäße Verfahren erlaubt ein effektives Training einer Osteosynthese einer Symphysenruptur. Die Schritte des Anlegens der kranialen Platte an das erste Schambein und des Anlegens der posterioren Platte an das erste Schambein können in beliebiger Reihenfolge, vorzugsweise auch simultan, erfolgen. In analoger Weise können auch das Befestigen der kranialen Platte und das Befestigen der posterioren Platte an dem ersten Schambein in beliebiger Reihenfolge, vorzugsweise simultan erfolgen. Das Einstellen des Symphysenabstands erfolgt vorzugswiese nach dem Befestigen der posterioren Platte und/oder der kranialen Platte an dem ersten Schambein des Trainingskörpers.

In einer ersten bevorzugten Weiterbildung weist das Verfahren ferner auf; Anlegen eines Positioniervorsprungs des medizinischen Implantats an das erste Schambein von einer der Symphyse des Trainingskörpers zugewandten Seite, wobei das Anlegen des Positioniervorsprungs vor dem Befestigen der kranialen Platte und/oder der posterioren Platte an dem ersten Schambein durchgeführt wird. Vorzugsweise wird bei dem erfindungsgemäßen Verfahren zunächst das medizinische Implantat an dem ersten Schambein befestigt, wobei eine korrekte Positionierung des Implantats durch den an das erste Schambein angelegten Positioniervorsprung sichergestellt wird. So kann das Implantat zunächst an dem ersten Schambein korrekt positioniert und befestigt werden, bevor der Symphysenabstand eingestellt und das Implantat bzw. die posteriore Platte und die kraniale Platte auch an dem zweiten Schambein befestigt werden. Das Verfahren ist gegenüber üblichen Trainingsverfahren deutlich leichter zu erlernen.

Ausführungsformen der Erfindung werden nun nachfolgend anhand der Zeichnungen beschrieben. Diese sollen die Ausführungsformen nicht notwendigerweise maßstäblich darstellen, vielmehr sind die Zeichnungen, wenn dies zur Erläuterung dienlich ist, in schematisierter und/oder leicht verzerrter Form ausgeführt. Im Hinblick auf Ergänzungen der aus den Zeichnungen unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in den Zeichnungen sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, den Zeichnungen und/oder den Ansprüchen offenbarten Merkmale. Die allgemeine Idee der Erfindung ist nicht beschränkt auf die exakte Form oder das Detail der im Folgenden gezeigten und beschriebenen bevorzugten Ausführungsformen oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein. Der Einfachheit halber sind nachfolgend für identische oder ähnliche Teile oder Teile mit identischer oder ähnlicher Funktion gleiche Bezugszeichen verwendet.

Weitere Vorteile, Merkmale und Einzelheiten der Erfindung ergeben sich aus der nachfolgenden Beschreibung der bevorzugten Ausführungsform sowie anhand der Zeichnungen, diese zeigen in:
- Figur 1: ein medizinisches Implantat in einer ersten Ausführungsform;
- Figur 2: das medizinische Implantat gemäß der ersten Ausführungsform, das an ein Modell eines menschlichen Hüftknochens angelegt ist;
- Figur 3: ein medizinisches Implantat in einer zweiten Ausführungsform; und in
- Figur 4: eine illustrierende Darstellung des erfindungsgemäßen Verfahrens.

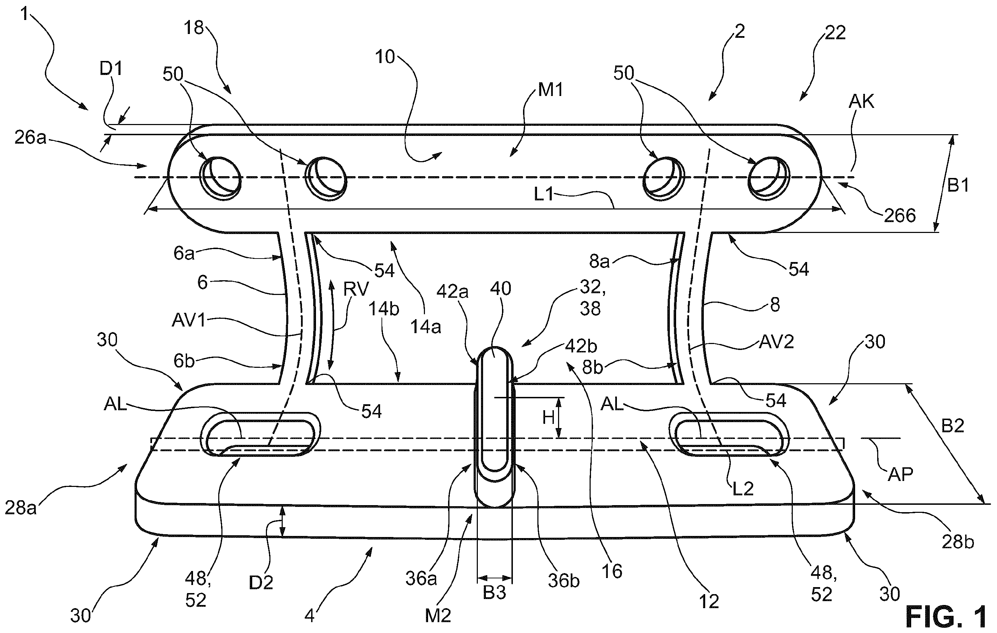

Figur 1 zeigt ein medizinisches Implantat 1 zur Osteosynthese von Symphysenrupturen am menschlichen Becken, das eine kraniale Platte 2 und eine posteriore Platte 4 aufweist. Die kraniale Platte 2 und die posteriore Platte 4 sind durch einen ersten Verbindungsarm 6 und einen zweiten Verbindungsarm 8 miteinander verbunden. Zum Anlegen an ein erstes Schambein 20 und ein zweites Schambein 24 eines zu behandelnden Patienten (nicht dargestellt), weist die kraniale Platte 2 eine kraniale Anlagefläche 10 auf. In analoger Weise weist die posteriore Platte 4 eine posteriore Anlagefläche 12 zum Anlegen an die Schambeine 20, 24 auf.

Die Verbindungsarme 6, 8 sind winklig ausgebildet, sodass die kraniale Anlagefläche 10 der kranialen Platte 2 und die posteriore Anlagefläche 12 der posterioren Platte 4 winklig zueinander angeordnet sind. In diesem Ausführungsbeispiel schließen die posteriore Anlagefläche 12 und die kraniale Anlagefläche 10 einen Winkel von etwa 90° ein. Eine winklige Anordnung der Anlageflächen 10, 12 kann aber auch durch bogenförmige, mehrachsig gekrümmte oder mehrfach geknickte Verbindungsarme 6, 8 erreicht werden. Der erste Verbindungsarm 6 erstreckt sich von einer der posterioren Platte 4 zugewandten Seitenfläche 14a der kranialen Platte 2 zu einer der kranialen Platte 2 zugewandten Seitenfläche 14b der posterioren Platte 4. Der zweite Verbindungsarm 8 erstrecken sich analog zwischen der Seitenfläche 14a der kranialen Platte 2 und der Seitenfläche 14b der posterioren Platte 12. Der erste Verbindungsarm 6 ist winklig ausgebildet, sodass ein erster Armabschnitt 6a im Wesentlichen senkrecht zu einem zweiten Armabschnitt 6b ausgebildet ist, wobei der erste Armabschnitt 6a mit der kranialen Platte 10 und der zweite Armabschnitt 6b mit der posterioren Platte 12 verbunden ist. Die Abschnitte 6a, 6b gehen rechtwinklig ineinander über. In analoger Weise sind auch ein erster Armabschnitt 8a und ein zweiter Armabschnitt 8b des zweiten Verbindungsarms 8 mit der kranialen Platte 10 und der posterioren Platte 12 verbunden.

Der erste Verbindungsarm 6 und der zweite Verbindungsarm 8 sind hier im Wesentlichen symmetrisch ausgebildet. Die Verbindungsarme 6, 8 weisen quer zu einer Verbindungsrichtung RV einen im Wesentlichen rechteckigen Querschnitt mit abgerundeten Ecken auf. Die Verbindungsrichtung ist, bedingt durch die rechtwinklige Anordnung der Armabschnitte 6a, 6b, 8a, 8b, gekrümmt bzw. geknickt und verläuft entlang der Verbindungsarme 6, 8. Die Verbindungsarme 6, 8 erstrecken sich seitlich von der kranialen Platte 10 und der posterioren Platte 12, wobei eine Längsachse AV1 des ersten Verbindungsarms 6 senkrecht zu einer Längsachse AK der kranialen Platte 2 ist. Zudem ist die Längsachse AV1 des ersten Verbindungsarms 6 im Wesentlichen rechtwinklig zu einer Längsachse AP der posterioren Platte 4. In analoger Weise ist auch eine Längsachse AV 2 des zweiten Verbindungsarms 8 im Wesentlichen rechtwinklig zur Längsachse AK der kranialen Platte 2 und zur Längsachse AP der posterioren Platte 4. Die Längsachsen AV1, AV2, AK, AP kennzeichnen die jeweils größte geometrische Erstreckung der Verbindungsarme 6, 8, der kranialen Platte 2 und der posterioren Platte 4.

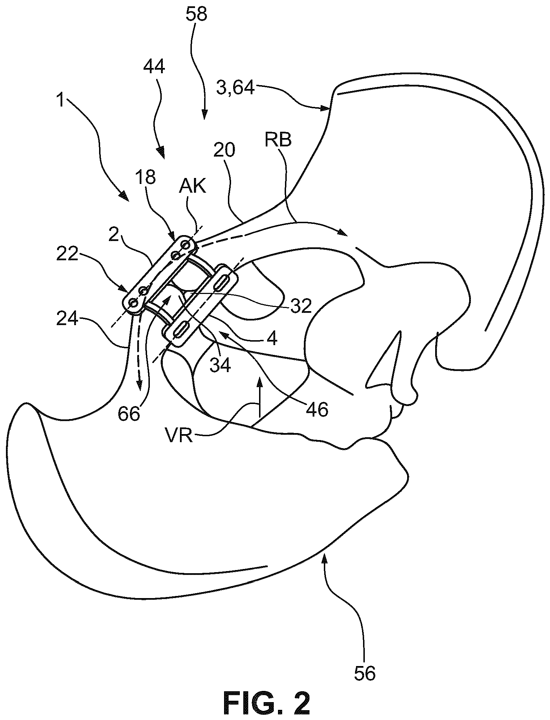

Die Verbindungsarme 6, 8 sind als außermittige Verbindungsarme 6, 8 ausgebildet. So ist der erste Verbindungsarm 6 beabstandet zu einer Mitte M1 der kranialen Platte 2 mit der kranialen Platte 2 verbunden und beabstandet zu einer Mitte M2 der posterioren Platte 4 mit der posterioren Platte 4 verbunden. In analoger Weise ist auch der zweite Verbindungsarm 8 beabstandet zur ersten Mitte M1 der kranialen Platte 2 und zur zweiten Mitte M2 der posterioren Platte 4 mit der kranialen Platte 2 und der posterioren Platte 4 verbunden. Zwischen den Verbindungsarmen 6, 8 ist so ein Sichtfenster 16 gebildet, welches das Ausrichten des medizinischen Implantats 1 im Rahmen der Osteosynthese einer Symphysenruptur erleichtert. Eine Symphyse 34 des zu behandelnden Patienten wird nicht durch die Verbindungsarme 6, 8 verdeckt. In diesem Ausführungsbeispiel ist das medizinische Implantat 1 spiegelsymmetrisch ausgebildet, wobei sich die nicht dargestellte Symmetrieebene durch die Mitte M1 der kranialen Platte 2 und die Mitte M2 der posterioren Platte 4 erstreckt. Ein erster Implantatabschnitt 18 zum Anlegen an ein erstes Schambein 20 (Figur 2) und ein zweiter Implantatabschnitt 22 zum Anlegen an ein zweites Schambein 24 sind im Wesentlichen identisch ausgebildet. Wenn beispielsweise eine Osteosynthese multipler Brüche (die eine Symphysenruptur umfassen) behandelt werden sollen, kann das medizinische Implantat 1 aber auch asymmetrisch ausgebildet sein. Beispielsweise kann der erste Implantatabschnitt 18 in Beckenringrichtung RB länger ausgebildet sein als der zweite Implantatabschnitt 22, um so ein Verbinden von Bruchstücken des ersten Schambeins 20 zu ermöglichen.

Die kraniale Platte 2 ist als ebene Platte ausgebildet, wobei die Längsachse AK der kranialen Platte 2 gerade ist. Die Längsachse AK definiert eine Längsrichtung der kranialen Platte 2. Zwei Enden 26a, 26b der kranialen Platte 2, die die kraniale Platte 2 in dieser Längsrichtung begrenzen, sind abgerundet. Die kraniale Anlagefläche 10 der kranialen Platte 2 ist im Wesentlichen rechteckig mit zwei halbkreisförmigen Abschnitten an den Enden 26a und 26b.

Die posteriore Platte 4 weist ebenfalls eine gerade Längsachse AP auf, die eine Längsrichtung der posterioren Platte 4 definiert. Die Anlagefläche 12 der posterioren Platte 4 ist im Wesentlichen rechteckig, wobei die posteriore Platte 4 in Längsrichtung begrenzende Enden 28a, 28b abgerundete Ecken 30 aufweisen. Zwischen den Ecken 30 ist die posteriore Anlagefläche 12 durch Geraden begrenzt. Im Gegensatz zur kranialen Platte 2 sind die Enden 28a, 28b der posterioren Platte 4 in diesem Ausführungsbeispiel also nicht halbkreisförmig ausgebildet.

Die Enden 26a, 26b der kranialen Platte 2 stehen in Längsrichtung der kranialen Platte 2 von den Verbindungsarmen 6, 8 hervor. Da sich die Längsachse AK der kranialen Platte 2 im Wesentlichen entlang einer Beckenringrichtung RB erstreckt, stehen die Enden 26a, 26b auch in der Beckenringrichtung RB von den Verbindungsarmen 6,8 hervor. Die Verbindungsarme 6, 8 sind nicht mit den Enden 26a, 26b verbunden, sondern münden zwischen der Mitte M1 und den Enden 26a, 26b in die kraniale Platte 2.

Die Enden 28a, 28b der posterioren Platte 4 stehen analog in Längsrichtung der posterioren Platte 4 von den Verbindungsarmen 6, 8 hervor, sodass die Verbindungsarme 6, 8 zwischen der Mitte M2 und den Enden 28a, 28b der posterioren Platte 4 in die posteriore Platte 4 münden. Auch die Längsachse AP der posterioren Platte 4 erstreckt sich im Wesentlichen in Beckenringrichtung RB sodass die Enden 28a, 28b der posterioren Platte 28a, 28b ebenfalls in Beckenringrichtung RB von den Verbindungsarmen 6, 8 hervorstehen.

Es kann jedoch auch vorgesehen sein, dass Verbindungsarme 6, 8 in die Enden 26a, 26b der kranialen Platte 2 und/oder die Enden 28a, 28b der posterioren Platte 4 münden. Ferner können die Verbindungsarme 6, 8 mit der Längsachse AK der kranialen Platte 2 und/oder der Längsachse AP der posterioren Platte 4 einen von 90° verschiedenen Winkel einschließen. So können die Verbindungsarme 6,8 beispielsweise auch V-förmig angeordnet sein.

Eine Länge L1 der kranialen Platte 2 wird entlang der Längsachse AK und eine Länge L2 der posterioren Platte 4 wird entlang der Längsachse AP ermittelt. Die Länge L1 der kranialen Platte 2 ist im Wesentlichen identisch zur Länge L2 der posterioren Platte 4. Eine Breite B1 der kranialen Platte 2 wird quer zur Längsachse AK ermittelt. In analoger Weise wird eine Breite B2 der posterioren Platte 4 quer zur Längsachse AP der posterioren Platte 4 bestimmt. In diesem Ausführungsbeispiel ist die Breite B1 der kranialen Platte 2 geringer als die Breite B 2 der posterioren Platte 4. So ist die kraniale Anlagefläche 10 der kranialen Platte 2 kleiner als die posteriore Anlagefläche 12 der posterioren Platte 4. Eine Dicke D1 der kranialen Platte 2, die quer zur kranialen Anlagefläche 10 und quer zur Längsachse AK der kranialen Platte 2 bestimmt wird, ist im Wesentlichen identisch zu einer Dicke D2 der posterioren Platte 4, gemessen quer zur posterioren Anlagefläche 12 und quer zur Längsachse AP der posterioren Platte 4. Es kann aber auch vorgesehen sein, dass sich die Dicken D1, D2 der kranialen Platte 2 und der posterioren Platte 4 unterscheiden. So kann beispielsweise eine Steifigkeit der Platten 2, 4 gegenüber Verformung an nach dem Implantieren auftretende Belastungen angepasst werden, um so ein Implantatversagen zu verhindern.

An der posterioren Platte 4 ist ein Positioniervorsprung 32 ausgebildet, der sich von der posterioren Platte 4 quer zur Beckenringrichtung RB erstreckt. Der Positioniervorsprung 32 ist zum Eingreifen in die Symphyse 34 (Figur 2) ausgebildet. Hier erstreckt sich der Positioniervorsprung 32 im Wesentlichen senkrecht von der posterioren Platte 4, sodass Seitenwände 46a, 46b des Positioniervorsprungs 32 im Wesentlichen senkrecht zur posterioren Anlagefläche 12 angeordnet sind. Der Positioniervorsprung 32 ist als langgestreckter Steg 38 ausgebildet, der sich über die gesamte Breite B2 der posterioren Platte 4 erstreckt. Alternativ kann der Positioniervorsprung aber vorzugsweise auch als sich von einer Anlagefläche 10, 12 erstreckender Zylinder, Kegel oder Keil ausgebildet sein.

Der Positioniervorsprung 32 ist mittig an der posterioren Platte 4 angeordnet und erstreckt sich von deren zweiter Mitte M2. Eine Stirnseite 40 des Positioniervorsprungs 32 ist stumpf ausgebildet, weist also keine scharfen Kanten auf. Übergänge 42a, 42b der Stirnseite 40 in die Seitenwände 46a, 46b des Positioniervorsprungs 32 sind abgerundet ausgebildet, sodass eine Gefahr beim Einsetzen des Positioniervorsprungs in die Symphyse 34 umliegendes Gewebe zu verletzen, minimiert ist. Hier ist die Stirnseite 40 im Wesentlichen parallel zur posterioren Anlagefläche 12. Es kann jedoch auch vorgesehen sein, dass Stirnseite 40 eine (vorzugsweise abgerundete) Spitze bildet oder kuppelförmig bzw. bogenförmig ausgebildet ist. Alternativ oder zusätzlich kann ein Positioniervorsprung 32 auch an der kranialen Platte 2 vorgesehen sein.

Eine Vorsprungshöhe H des Positioniervorsprungs 32 wird quer zur Anlagefläche 12 ermittelt. Liegt die kraniale Platte 2 von einer kranialen Seite 44 an dem ersten Schambein 20 und dem zweiten Schambein 24 an und liegt die posteriore Platte 4 von einer posterioren Seite 46 an dem ersten und zweiten Schambein 20, 24 an, ist die Vorsprungshöhe H ein Maß dafür, wie weit der Positioniervorsprung 32 in die Symphyse 34 eingreift. Vorzugsweise ist eine Vorsprungsbreite B3 des Positioniervorsprungs 32, die in Beckenringrichtung RB bestimmt wird, an die Breite einer menschlichen Symphyse angepasst. Im Rahmen einer Osteosynthese einer Symphysenruptur wird das korrekte Positionieren des medizinischen Implantats 1 und ein Einstellen des Symphysenabstands zwischen dem ersten Schambein 20 und dem zweiten Schambein 24 erheblich erleichtert. So kann der erste Implantatabschnitt 18 zunächst an das erste Schambein 20 angelegt werden, wobei der Positioniervorsprung 32, der in die Symphyse 34 eingreift, eine optimale Position des medizinischen Implantats 1 in Beckenringrichtung RB gewährleistet. Anschließend kann der erste Implantatabschnitt 18 an dem ersten Schambein 20 befestigt werden. Das medizinische Implantat 1 ist dann fest mit dem ersten Schambein 20 verbunden. Anschließend kann der Symphysenspalt zwischen dem ersten Schambein 20 und dem zweiten Schambein 24 eingestellt werden, indem das zweite Schambein 24 auf einer dem ersten Schambein 20 gegenüberliegenden Seite mit dem Positioniervorsprung 32 in Kontakt gebracht wird. Zum Verbinden der beiden Schambeine 20, 24 wird anschließend der zweite Implantatabschnitt 22 mit dem zweiten Schambein 24 verbunden.

Zum Verbinden des medizinischen Implantats 1 mit dem ersten Schambein 20 und dem zweiten Schambein 24 weist die posteriore Platte 4 erste Verbindunglöcher 48 auf. Ferner weist die kraniale Platte 2 zweite Verbindunglöcher 50 auf. Im Rahmen der Osteosynthese könne erste Verbindungsmittel (nicht dargestellt) durch die ersten Verbindunglöcher 48 der posterioren Platte 4 und zweite Verbindungsmittel (nicht dargestellt) durch die zweiten Verbindungslöcher 50 der kranialen Platte 2 geführt werden. Solche Verbindungsmittel sind üblicherweise medizinische Schrauben, Nägel und/oder Stifte. Ein Kopfabschnitt der Verbindungsmittel liegt dann auf einer den Anlageflächen 10, 12 gegenüberliegenden Seite der Platten 2,4 an und spannt die posteriore Platte 4 bzw. die kraniale Platte 2 gegen das erste Schambein 20 oder das zweite Schambein 24.

Die posteriore Platte 4 weist in den gezeigten Ausführungsformen zwei erste Verbindungslöcher 48 auf, die als Ovallöcher 52 ausgebildet sind. Es soll verstanden werden, dass die posteriore Platte 4 auch nur ein erstes Verbindungsloch 48 oder mehr als zwei erste Verbindungslöcher 48 aufweisen kann. Ferner müssen nicht alle ersten Verbindungslöcher 48 als Ovallöcher 52 ausgebildet sein. Längsachsen AL der Ovallöcher 52 liegen auf der Längsachse AP der posterioren Platte 4. Die Längsachse AL der Ovallöcher 52 kennzeichnet eine maximale lichte Weite der Ovallöcher 52 quer zu derjenigen Richtung, in der die Befestigungsmittel durch die Ovallöcher 52 geführt werden. Hier sind die Ovallöcher 52 aus zwei halbkreisförmigen Abschnitten gebildet, die mittels zur Längsachse AL der Ovallöcher 52 paralleler Abschnitte verbunden sind. Verbindungslöcher 48 mit einer solchen Querschnittsgeometrie werden auch häufig als Langlöcher bezeichnet. Die Ovallöcher 52 liegen auf der Längsachse AP der posterioren Platte 4, die sich im Wesentlichen entlang der Beckenringrichtung RB erstreckt, sodass sich die Längsachse AL der Ovallöcher 52 ebenfalls im Wesentlichen entlang der Beckenringrichtung RB erstreckt. Die Ovallöcher 52 erleichtern ein Bohren von Befestigungslöchern (nicht dargestellt) in das erste Schambein 20 und das zweite Schambein 24, die im Rahmen der Osteosynthese gebohrt werden müssen, um ein Befestigen des Implantats 1 zu ermöglichen. Ein nicht dargestellter Bohrer muss beim Bohren nicht senkrecht zur posterioren Seite 46 geführt werden, sondern kann auch winklig angesetzt werden.

Die kraniale Platte 2 weist insgesamt vier zweite Befestigungslöcher 50 auf, die hier als Rundlöcher ausgebildet sind. Quer zu derjenigen Richtung, in der Befestigungsmittel durch die Löcher 50 geführt werden (Durchgangsrichtung), haben die Befestigungslöcher 50 einen kreisrunden Querschnitt. Es kann jedoch auch vorgesehen sein, dass einige oder alle der zweiten Verbindunglöcher 50 als Ovallöcher 52 ausgebildet sind. Im Rahmen einer Osteosynthese kann die Zugänglichkeit von der kranialen Seite 44 meist besser gewährleistet werden als eine Zugänglichkeit der posterioren Seite 46, weshalb zumindest die posteriore Platte 4 vorzugsweise Ovallöcher 52 aufweist. Rundlöcher hingegen verhindern in vorteilhafter Weise ein Verrutschen der Befestigungsmittel. Vorzugsweise können die ersten Befestigungslöcher 48 und/oder die zweiten Befestigungslöcher 50 in Durchgangsrichtung konisch ausgebildet sein.

Die ersten Befestigungslöcher 48 und/oder die zweiten Befestigungslöcher 50 sind vorzugsweise im Bereich der Verbindungsarme 6, 8 angeordnet. So erstreckt sich die Längsachse AV1 des ersten Verbindungsarm 6 an der kranialen Platte 2 vorzugsweise zwischen den Befestigungslöchern 50 des ersten Implantatabschnitts 18. In analoger Weise erstreckt sich die zweite Längsachse AV2 des zweiten Verbindungsarms 8 an der kranialen Platte 2 zwischen den Befestigungslöchern 50 des zweiten Implantatabschnitts 20. An der posterioren Platte 4 erstreckt sich die erste Längsachse AV1 des ersten Verbindungsarms 6 und/oder die zweite Längsachse AV2 des zweiten Verbindungsarms 8 durch die Langlöcher 52 am ersten Implantatabschnitt 18 bzw. am zweiten Implantatabschnitt 22.

Vorzugsweise sind die ersten Verbindungslöcher 48 und/oder die zweiten Verbindunglöcher 50 in einer Breitenrichtung, die parallel zur ersten und zweiten Breite B1, B2 verläuft, mittig an der kranialen Platte 2 bzw. der posterioren Platte 4 angeordnet.

Ein Querschnitt der Verbindungsarme 6, 8 ist so gewählt, dass die Verbindungsarme 6, 8 eine geringere Steifigkeit aufweisen als die kraniale Platte 2 und die posteriore Platte 4. Dadurch können die Verbindungsarme 6, 8 plastisch verformt werden, um eine Ausrichtung der kranialen Anlagefläche 10 zur posterioren Anlagefläche 12 zu ändern. So kann die in Figur 1 dargestellte rechtwinklige Anordnung der kranialen Anlagefläche 10 zur posterioren Anlagefläche 12 durch Aufbiegen der Verbindungsarme 6, 8 geändert werden. Nach dem Aufbiegen schließen die kraniale Anlagefläche 10 und die posteriore Anlagefläche 12 einen Winkel von größer 90° ein. In analoger Weise kann das medizinische Implantat 1 auch zusammengebogen werden, sodass die kraniale Anlagefläche 10 und die posteriore Anlagefläche 12 einen Winkel kleiner 90° einschließen. Es soll verstanden werden, dass die Anlageflächen 10, 12 vor dem Aufbiegen bzw. dem Zusammenbiegen auch einen zu 90° verschiedenen Winkel aufweisen können. Beim plastischen Verformen eines oder beider Verbindungsarme 6, 8, ändert sich die Form der Längsachse AV1 des ersten Verbindungsarms 6 und/oder der zweiten Verbindungsachse AV2 des zweiten Verbindungsarms 8. Die kraniale Platte 2 und die posteriore Platte 4, deren Steifigkeit bei der gezeigten einstückigen Ausgestaltung im Wesentlichen durch die Breite B1, B2 und die Dicken D1, D2 festgelegt ist, werden nicht verformt und bleiben eben. Die plastisch verformbaren Verbindungsarme 6, 8 ermöglichen so ein Anpassen des medizinischen Implantats 1 an die Gestalt des ersten Schambeins 20 und des zweiten Schambeins 24. Vorzugsweise sind die Verbindungsarme 6, 8 werkzeuglos verformbar.

Beim plastischen Verformen werden die Längsachsen AV1, AV2 der Verbindungsarme 6, 8 gekrümmt. Es kann jedoch auch vorgesehen sein, dass Übergangsbereiche 54 der Verbindungsarme 6, zu den Platten 2, 4 verformt werden, während die Gestalt der Verbindungsarm 6, 8 im Wesentlichen konstant bleibt. Eine Querschnittsfläche (nichts dargestellt) der Verbindungsarme 6, 8 quer zu deren Längsachsen AV1, AV2 ist entsprechend so gewählt, dass die Steifigkeit der Verbindungsarme 6,8 geringer ist als die Steifigkeit der Platten 2, 4. Ferner beeinflusst die Querschnittsgeometrie der Verbindungsarme 6, 8 die Steifigkeit der Verbindungsarme, wobei runde, ovale, rechteckige, quadratische, I-förmige, T-förmige, L-förmige und/oder ringförmige Querschnittsformen der Verbindungsarme bevorzugt sind. Es kann aber auch vorgesehen sein, dass wenigstens einer der Verbindungsarme 6,8 einen nicht dargestellten Verformungsabschnitt aufweist, der aus einem leichter verformbaren Material gefertigt ist als die Platten 2,4. Beispielsweise können die Verbindungsarme 6, 8 abschnittsweise aus Titan gebildet sein, während die posteriore Platte 4 und die kraniale Platte 2 aus Stahl gebildet sind.

Figur 2 verdeutlicht die Anordnung des medizinischen Implantats 1 im, Rahmen der Osteosynthese einer Symphysenruptur am menschlichen Becken 3, wobei anstelle eines echten menschlichen Beckens 3 ein Trainingskörper 64 gezeigt ist, der die natürliche Gestalt eines menschlichen Beckens 3 detailgetreu nachbildet. Die kraniale Platte 2 wird von der kranialen Seite 44 an das erste Schambein 20 und das zweite Schambein 24 angelegt. Die kraniale Platte 2 überbrückt so von der kranialen Seite 44 die Symphyse 34. Auf die Schambeine 20, 24 aufgebrachte Kräfte können von der kranialen Platte 2 vom ersten Schambein 20 auf das zweite Schambein 24 und umgekehrt übertragen werden. Die posteriore Platte 4 wird von der posterioren Seite 46 an die Schambeine 20, 24 angelegt und überbrückt ebenfalls die Symphyse 34. Der Positioniervorsprung 32 greift beim korrekten Positionieren des medizinischen Implantats 1 am Becken 3 in die Symphyse 34 ein.

In der in Figur 2 gezeigten ersten Ausführungsform sind die kraniale Platte 2 und die posteriore Platte 4 gerade und ebene Platten. Dennoch erstrecken sich die posteriore Platte 4 und die kraniale Platte 4 im Wesentlichen entlang der Beckenringrichtung RB. Der als Steg 38 ausgebildete Positioniervorsprung 32 erstreckt sich in einer vertikalen Richtung VR von einer inferioren Seite 56 zu einer superioren Seite 58 des Beckens 3. Senkrecht zur posterioren Platte 4 erstreckt sich der Positioniervorsprung 32 nicht vollständig von der posterioren Anlagefläche 12 durch die Symphyse 34.

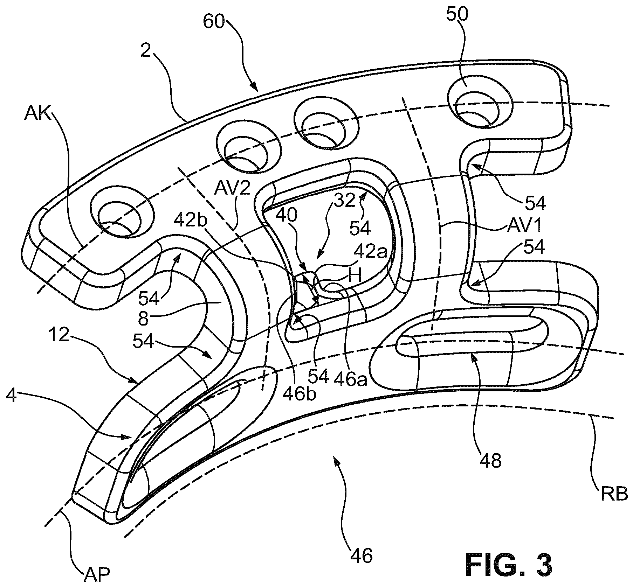

Figur 3 verdeutlicht eine zweite Ausführungsform des medizinischen Implantats 1. In der zweiten Ausführungsform ist die Gestalt des medizinischen Implantats 1 weiter an eine natürliche Gestalt des menschlichen Beckens 3 angepasst. Im Gegensatz zu Figur 1, die eine den Schambeinen 20, 24 zugewandte Seite des medizinischen Implantat 1 zeigt, verdeutlicht Figur 3 eine Ansicht auf eine den Schambeinen 20, 24 abgewandte Seite des medizinischen Implantats 1.

Die zweite Ausführungsform gemäß Figur 3 ist im Wesentlichen analog zur ersten Ausführungsform gemäß den Figuren 1 und 2 ausgebildet, wobei gleiche Abschnitte und Komponenten mit gleichen Bezugszeichen versehen sind. Im Gegensatz zur ersten Ausführungsform sind die kraniale Platte 2 und die posteriore Platte 4 gemäß dem zweiten Ausführungsbeispiel nicht als gerade Platten ausgebildet, sondern gekrümmt. Die kraniale Platte 2 ist eben gekrümmt und die kraniale Anlagefläche 10 ist eine ebene Fläche. Die Längsachse AK der kranialen Platte 2 ist in einer Transversalebene, die im Wesentlichen parallel zur Bildebene in Figur 3 ist, konvex gekrümmt, sodass eine Ausbauchung 60 der kranialen Platte 2 von der posterioren Seite 46 abgewandt ist. Ein Hervorstehen der kranialen Platte 2 über die Schambeine 20, 24 nach erfolgter Osteosynthese wird so bei gleichzeitiger Maximierung der Kontaktfläche minimiert.

Auch die posteriore Platte 4 ist gekrümmt ausgebildet. Die Längsachse AP der posterioren Platte 4 ist konvex. Eine Ausbauchung 62 der posterioren Platte 4 weist von der posterioren Seite 46 weg, also in die gleiche Richtung wie die Ausbauchung 60 der kranialen Platte 2. Die posteriore Anlagefläche 12 ist im Gegensatz zum ersten Ausführungsbeispiel nicht eben, sondern bogenförmig gekrümmt ausgebildet. Hier ist die posteriore Platte 4 nur einachsig gekrümmt (nur die Längsachse AP der posterioren Platte 4 ist gekrümmt). Es kann aber auch vorgesehen sein, dass die posteriore Platte mehrachsig gekrümmt ausgebildet ist. Durch die Krümmung ist die posteriore Anlagefläche 12 optimal an die Form der Schambeine 20, 24 des Beckens 3 angepasst. Alternativ zur gezeigten Ausgestaltung kann vorgesehen sein, dass die posteriore Analagefläche 12 gekrümmt ist, während einer der posterioren Anlagefläche 12 gegenüberliegende Fläche der posterioren Platte 12 eben ist.

Ferner weisen die Verbindungsarme 6, 8 der zweiten Ausführungsform des medizinischen Implantats 1 im Vergleich zur ersten Ausführungsform einen größeren Querschnitt auf. Ein Widerstand der Verbindungsarme 6,8 gegenüber Verformung ist erhöht, sodass auf die Platten 2, 4 aufgebrachte Kräfte besser zwischen den Platten 2, 4 übertragen werden können. Allerdings ist zum Anpassen des medizinischen Implantats 1 an das Becken 3 ein größerer Kraftaufwand notwendig. Die zwischen den Verbindungsarmen 6, 8 und den Platten 2,4 ausgebildeten Übergänge 54 sind verrundet, wodurch eine Stabilität des medizinischen Implantats 1 verbessert und eine Beeinträchtigung von umgebendem Gewebe verhindert werden kann.

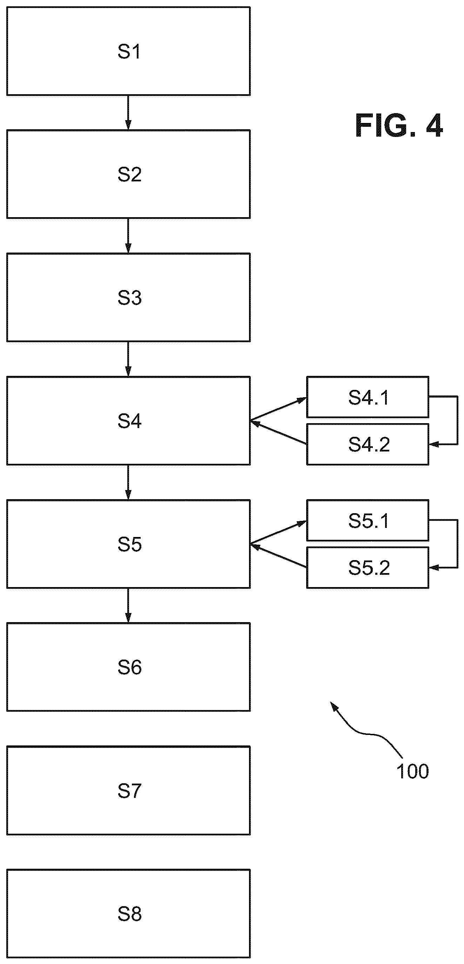

Figur 4 illustriert ein Verfahren 100 zur Osteosynthese einer Symphysenruptur an einem Trainingskörper 64 zu Trainingszwecken mittels eines medizinischen Implantats 1. In einem ersten Schritt S1 wird die kraniale Platte 2 des medizinischen Implantats 1 an das erste Schambein 20 des Trainingskörpers 64 angelegt. Das Anlegen erfolgt von der kranialen Seite 44.

In einem zweiten Schritt S2 wird die posteriore Platte 4 des medizinischen Implantats 1 an das erste Schambein 20 des Trainingskörpers 64 angelegt, wobei das Anlegen von der posterioren Seite 46 erfolgt. Es soll verstanden werden, dass die Schritte S1 und S2 zeitgleich oder in vertauschter Reihenfolge ausgeführt werden können.

In einem dritten Schritt S3 wird der Positioniervorsprung 32 des medizinischen Implantats 1 von einer der Symphyse 34 des Trainingskörpers 64 zugewandten Seite 66 an das erste Schambein 20 angelegt. Hierdurch wird eine optimale Positionierung des ersten Implantatabschnitts18 zum ersten Schambein 20 sichergestellt.

Anschließend wird in einem vierten Schritt S4 die kraniale Platte 2 an dem ersten Schambein 20 befestigt. Hierfür können in einem ersten Teilschritt S4.1 zunächst durch die zweiten Verbindunglöcher 50 Befestigungslöcher (nicht dargestellt) in das erste Schambein 20 gebohrt werden. Anschließend werden in einem zweiten Teilschritt S4.2 Verbindungsmittel durch die zweiten Verbindungslöcher 50 geführt und in die zuvor gebohrten Befestigungslöcher eingesetzt, wodurch die kraniale Platte 2 am ersten Schambein 18 befestigt wird.

In einem fünften Schritt S5 wird die posteriore Platte 4 an dem ersten Schambein 20 befestigt. Analog zum vierten Schritt S4 können auch im fünften Schritt S5 in einem ersten Teilschritt S5.1 zunächst durch die ersten Verbindungslöcher 48 Befestigungslöcher (nicht dargestellt) in das erste Schambein 20 gebohrt werden. In einem zweiten Teilschritt S5.2 können dann Verbindungsmittel (nicht dargestellt) durch die ersten Verbindungslöcher 48 geführt und in die Befestigungslöcher eingesetzt werden, um die posteriore Platte 4 an dem ersten Schambein 20 zu befestigen. Gegebenenfalls kann der erste Teilschritt S5.1 auch entfallen und die Verbindungsmittel, beispielsweise Schrauben, unmittelbar eingesetzt werden. Die Schritte S4 und S5 können in beliebiger Reihenfolge, beispielsweise parallel oder in umgekehrter Reihenfolge, durchgeführt werden. So können beispielsweise zunächst die Teilschritte S4.1, und S5.1 durchgeführt werden bevor die posteriore Platte 4 und die kraniale Platte 2 mittels der Befestigungsmittel an dem ersten Schambein 20 befestigt werden (S 5.2, S 4.2). Alternativ kann beispielsweise die posteriore Platte 4 vor der kranialen Platte 2 am ersten Schambein 20 befestigt werden.

In einem anschließenden sechsten Schritt S6 wird ein in Beckenringrichtung RB betrachteter Symphysenabstand zwischen dem ersten Schambein 20 und dem zweiten Schambein 24 eingestellt. Hierfür wird vorzugsweise das zweite Schambein 24 auf das erste Schambein 20 zubewegt, bis das erste Schambein 24 an dem Positioniervorsprung 32 anliegt. Der Positioniervorsprung 32 bzw. dessen Breite B3 geben den Symphysenabstand der Symphyse 34 vor. Das Ausrichten der Schambeine 20, 24 des Trainingskörpers 64 zueinander wird erleichtert.

In einem siebten Teilschritt S7 wird die kraniale Platte 2 mit dem zweiten Schambein 24 verbunden, wobei das zweite Schambein 20 zunächst an die kraniale Platte 2 angelegt wird. Der siebte Schritt S7 kann, analog zum vierten Schritt S4, Teilschritte aufweisen.

In einem achten Schritt S8 wird das zweite Schambein 24 an der posterioren Platte 4 befestigt, wobei das zweite Schambein 24 zunächst an die posteriore Platte 4 angelegt wird. Der achte Schritt S8 kann, analog zum fünften Schritt S5, Teilschritte aufweisen. Ferner können die Schritte S7 und S8 in beliebiger Reihenfolge durchgeführt werden.

## Patentansprüche

1. Medizinisches Implantat (1) zur Osteosynthese von Symphysenrupturen am menschlichen Becken, aufweisend
eine kraniale Platte (2), die dazu ausgebildet ist, von einer kranialen Seite (44) an einem ersten Schambein (20) und einem zweiten Schambein (24) eines zu behandelnden Patienten befestigt zu werden, um die Schambeine (20, 24) zu verbinden,
eine posteriore Platte (4), die dazu ausgebildet ist, von einer posterioren Seite (46) an dem ersten Schambein (20) und dem zweiten Schambein (24) des Patienten befestigt zu werden, um die Schambeine (20, 24) zu verbinden,
wobei die kraniale Platte (2) und die posteriore Platte (4) durch wenigstens einen ersten Verbindungsarm (6) miteinander verbunden sind, und
wobei das medizinische Implantat (1) ferner einen Positioniervorsprung (32) zum zumindest teilweisen Eingreifen in die Symphyse (34) des Beckens des zu behandelnden Patienten aufweist.

2. Medizinisches Implantat (1) nach Anspruch 1, wobei der erste Verbindungsarm (6) ein bezogen auf die posteriore Platte (4) und die kraniale Platte (2) außermittiger Verbindungsarm (6) ist.

3. Medizinisches Implantat (1) nach Anspruch 1 oder 2, ferner aufweisend einen zweiten Verbindungsarm (8), wobei der zweite Verbindungsarm (8) vorzugsweise ein bezogen auf die posteriore Platte (4) und die kraniale Platte (2) außermittiger Verbindungsarm (8) ist.

4. Medizinisches Implantat (1) nach Anspruch 3, wobei der Positioniervorsprung (32) zwischen dem ersten Verbindungsarm (6) und dem zweiten Verbindungsarm (8) angeordnet ist.

5. Medizinisches Implantat (1) nach einem der vorstehenden Ansprüche, wobei der Positioniervorsprung (32) mittig am Implantat (1), vorzugsweise in einer Symmetrieebene des Implantats (1), angeordnet ist, und/oder wobei der Positioniervorsprung (32) eine stumpfe Stirnseite (40) aufweist, und/oder wobei der Positioniervorsprung (32) ein quer zu einer Vorsprungshöhe (H) des Positioniervorsprungs (32) langgestreckter Steg (38) ist.

6. Medizinisches Implantat (1) nach einem der vorstehenden Ansprüche, wobei sich der Positioniervorsprung (32) im Wesentlichen senkrecht von einer Anlagefläche (10, 12) an der kranialen Platte (2) und/oder der posterioren Platte (4) des medizinischen Implantats (1) erstreckt.

7. Medizinisches Implantat (1) nach einem der vorstehenden Ansprüche, wobei der Positioniervorsprung (32) an der posterioren Platte (4) vorgesehen ist.

8. Medizinisches Implantat (1) nach einem der vorstehenden Ansprüche, wobei der Positioniervorsprung (32) eine Vorsprungshöhe (H) in einem Bereich von 1 mm bis 15 mm, vorzugsweise 2 mm bis 5 mm aufweist.

9. Medizinisches Implantat (1) nach einem der vorstehenden Ansprüche, wobei die posteriore Platte (4) erste Befestigungslöcher (48) aufweist, die zum Aufnehmen von ersten Verbindungsmitteln vorgesehen sind, wobei die kraniale Platte (2) zweite Befestigungslöcher (50) aufweist, die zum Aufnehmen von zweiten Verbindungsmitteln vorgesehen sind, und wobei die ersten Befestigungslöcher (48) oder die zweiten Befestigungslöcher (50) als Ovallöcher (52) ausgebildet sind, wobei eine Längsachse (AL) der Ovallöcher (52) vorzugsweise im Wesentlichen entlang einer Beckenringrichtung (RB) orientiert ist.

10. Medizinisches Implantat (1) nach einem der vorstehenden Ansprüche, wobei der Verbindungsarm (6,8) eine geringere Steifigkeit aufweist als die posteriore Platte (4) und die kraniale Platte (2), sodass eine Ausrichtung der posterioren Platte (4) relativ zur kranialen Platte (2) durch plastisches Verformen des Verbindungsarms (6, 8) einstellbar ist.

11. Medizinisches Implantat (1) nach Anspruch 1, wobei die kraniale Platte (2) in einer Transversalebene konvex ausgebildet ist und/oder wobei die posteriore Platte (4) in der Transversalebene konvex ausgebildet ist.

12. Medizinisches Implantat (1) nach einem der vorstehenden Ansprüche, wobei ein erster Implantatabschnitt (18) zur Befestigung an dem ersten Schambein (20) und ein zweiter Implantatabschnitt (22) zur Befestigung an dem zweiten Schambein (24) im Wesentlichen spiegelsymmetrisch zueinander ausgebildet sind, und/oder wobei eine Länge (L2) der posterioren Platte (4) und eine Länge (L1) der kranialen Platte (4) im Wesentlichen identisch sind.

13. Medizinisches Implantat (1) nach einem der vorstehenden Ansprüche, wobei der zumindest eine Verbindungsarm (6, 8) in einer Beckenringrichtung (RB) zu Enden (28a, 28b) der posterioren Platte (4) und/oder Enden (26a, 26b) der kranialen Platte (2) beabstandet angeordnet ist.

14. Verfahren (100) zur Osteosynthese einer Symphysenruptur an einem Trainingskörper (64) zu Trainingszwecken mittels eines medizinischen Implantats (1) gemäß einem der vorstehenden Ansprüche 1 bis 13, wobei der Trainingskörper (64) kein lebender menschlicher oder tierischer Körper ist, das Verfahren (100) aufweisend die Schritte:
- Anlegen (S1) einer kranialen Platte (2) des medizinischen Implantats (1) an einem ersten Schambein (20) des Trainingskörpers (64) von einer kranialen Seite (44);
- Anlegen (S2) einer posterioren Platte (4) des medizinischen Implantats (1) an einem ersten Schambein (20) des Trainingskörpers (64) von einer posterioren Seite (46);
- Befestigen (S4) der kranialen Platte (2) an dem ersten Schambein (20);
- Befestigen (S5) der posterioren Platte (4) an dem ersten Schambein (20);
- Einstellen eines Symphysenabstands (S6) zwischen dem ersten Schambein (20) und dem zweiten Schambein (24);
- Befestigen (S7) der kranialen Platte (2) an dem zweiten Schambein (24) und Befestigen (S8) der posterioren Platte (4) an dem zweiten Schambein (24) nach dem Einstellen des Symphysenabstands (S6).

15. Verfahren (100) nach Anspruch 14, ferner aufweisend den Schritt:
- Anlegen (S3) eines Positioniervorsprungs (32) des medizinischen Implantats (1) an das erste Schambein (20) von einer der Symphyse (34) des Trainingskörpers zugewandten Seite (66),
wobei das Anlegen (S3) des Positioniervorsprungs (32) vor dem Befestigen (S4, S5) der kranialen Platte (2) und/oder der posterioren Platte (4) an dem ersten Schambein (20) durchgeführt wird.

## Claims

1. A medical implant (1) for osteosynthesis of symphysis ruptures on the human pelvis, comprising
a cranial plate (2) configured to be attached to a first pubic bone (20) and a second pubic bone (24) of a patient to be treated from a cranial side (44) for connecting the pubic bones (20, 24),
a posterior plate (4) configured to be attached to the first pubic bone (20) and the second pubic bone (24) of the patient from a posterior side (46) for connecting the pubic bones (20, 24),
wherein the cranial plate (2) and the posterior plate (4) are connected to each other by at least one first connecting arm (6), and
wherein the medical implant (1) further comprises a positioning protrusion (32) for at least partially engaging the symphysis (34) of the pelvis of the patient to be treated.

2. The medical implant (1) according to claim 1, wherein the first connecting arm (6) is an off-center connecting arm (6) with respect to the posterior plate (4) and the cranial plate (2).

3. The medical implant (1) according to claim 1 or 2, further comprising a second connecting arm (8), wherein the second connecting arm (8) preferably is an off-center connecting arm (8) with respect to the posterior plate (4) and the cranial plate (2).

4. The medical implant (1) according to claim 3, wherein the positioning protrusion (32) is arranged between the first connecting arm (6) and the second connecting arm (8).

5. The medical implant (1) according to any of the preceding claims, wherein the positioning protrusion (32) is arranged centered on the implant (1), preferably on a symmetry plane of the implant (1), and/or wherein the positioning protrusion (32) has a blunt end face (40), and/or wherein the positioning protrusion (32) is a ridge (38) that is elongated transverse to a protrusion height (H) of the positioning protrusion (32).

6. The medical implant (1) according to any of the preceding claims, wherein the positioning protrusion (32) extends substantially perpendicular from a contact surface (10, 12) on the cranial plate (2) and/or the posterior plate (4) of the medical implant (1).

7. The medical implant (1) according to any of the preceding claims, wherein the positioning protrusion (32) is provided on the posterior plate (4).

8. The medical implant (1) according to any of the preceding claims, wherein the positioning protrusion (32) has a protrusion height (H) in a range from 1 mm to 15 mm, preferably 2 mm to 5 mm.

9. The medical implant (1) according to any of the preceding claims, wherein the posterior plate (4) comprises first mounting holes (48) provided for receiving first connecting means, wherein the cranial plate (2) comprises second mounting holes (50) provided for receiving second connecting means, and wherein the first mounting holes (48) or the second mounting holes (50) are oval holes (52), wherein a longitudinal axis (AL) of the oval holes (52) is preferably oriented substantially along a pelvic ring direction (RB).

10. The medical implant (1) according to any of the preceding claims, wherein the connecting arm (6, 8) has a lower rigidity than the posterior plate (4) and the cranial plate (2), so that an alignment of the posterior plate (4) relative to the cranial plate (2) can be adjusted by plastically deforming the connecting arm (6, 8).

11. The medical implant (1) according to claim 1, wherein the cranial plate (2) is convex in a transversal plane and/or wherein the posterior plate (4) is convex in the transversal plane.

12. The medical implant (1) according to any of the preceding claims, wherein a first implant section (18) for attachment to the first pubic bone (20) and a second implant section (22) for attachment to the second pubic bone (24) are configured substantially mirror-symmetrical to each other, and/or wherein a length (L2) of the posterior plate (4) and a length (L1) of the cranial plate (4) are substantially identical.

13. The medical implant (1) according to any of the preceding claims, wherein the at least one connecting arm (6, 8) is spaced apart in a pelvic ring direction (RB) from ends (28a, 28b) of the posterior plate (4) and/or ends (26a, 26b) of the cranial plate (2).

14. Method (100) for osteosynthesis of a symphysis rupture on a training body (64) for training purposes with a medical implant (1) according to any of the preceding claims 1 to 13, wherein the training body (64) is not a living human or animal body, the method (100) comprising the steps:
- placing (S1) a cranial plate (2) of the medical implant (1) to a first pubic bone (20) of the training body (64) from a cranial side (44);
- placing (S2) a posterior plate (4) of the medical implant (1) to a first pubic bone (20) of the training body (64) from a posterior side (46);
- attaching (S4) the cranial plate (2) to the first pubic bone (20);
- attaching (S5) the posterior plate (4) to the first pubic bone (20);
- adjusting a symphysis gap (S6) between the first pubic bone (20) and the second pubic bone (24);
- attaching (S7) the cranial plate (2) to the second pubic bone (24) and attaching (S8) the posterior plate (4) to the second pubic bone (24) after adjusting the symphysis gap (S6).

15. Method (100) according to claim 14, further comprising the step of:
- placing (S3) a positioning protrusion (32) of the medical implant (1) on the first pubic bone (20) from a side (66) facing the symphysis (34) of the training body,
wherein placing (S3) of the positioning protrusion (32) is performed before attaching (S4, S5) the cranial plate (2) and/or the posterior plate (4) to the first pubic bone (20).

## Revendications

1. Implant médical (1) pour l'ostéosynthèse des disjonctions symphisaires du bassin humain, présentant
une plaque crânienne (2) qui est configurée pour être fixée depuis un côté crânien (44) à un premier os pubien (20) et à un deuxième os pubien (24) d'un patient à traiter, afin de relier les os pubiens (20, 24),
une plaque postérieure (4) qui est configurée pour être fixée depuis un côté postérieur (46) au premier os pubien (20) et au deuxième os pubien (24) du patient, afin de relier les os pubiens (20, 24),
la plaque crânienne (2) et la plaque postérieure (4) étant reliées l'une à l'autre par au moins un premier bras de liaison (6), et
l'implant médical (1) présentant en outre une saillie de positionnement (32) destinée à s'engager au moins partiellement dans la symphyse (34) du bassin du patient à traiter.

2. Implant médical (1) selon la revendication 1, le premier bras de liaison (6) étant un bras de liaison (6) excentré par rapport à la plaque postérieure (4) et à la plaque crânienne (2).

3. Implant médical (1) selon la revendication 1 ou 2, présentant en outre un deuxième bras de liaison (8), le deuxième bras de liaison (8) étant de préférence un bras de liaison (8) excentré par rapport à la plaque postérieure (4) et à la plaque crânienne (2).

4. Implant médical (1) selon la revendication 3, la saillie de positionnement (32) étant disposée entre le premier bras de liaison (6) et le deuxième bras de liaison (8).

5. Implant médical (1) selon l'une des revendications précédentes, la saillie de positionnement (32) étant disposée au centre de l'implant (1), de préférence dans un plan de symétrie de l'implant (1), et/ou la saillie de positionnement (32) présentant une face frontale émoussée (40), et/ou la saillie de positionnement (32) étant une barrette (38) allongée transversalement par rapport à une hauteur de saillie (H) de la saillie de positionnement (32).

6. Implant médical (1) selon l'une des revendications précédentes, la saillie de positionnement (32) s'étendant sensiblement perpendiculairement à partir d'une surface d'appui (10, 12) sur la plaque crânienne (2) et/ou la plaque postérieure (4) de l'implant médical (1).

7. Implant médical (1) selon l'une des revendications précédentes, la saillie de positionnement (32) étant prévue sur la plaque postérieure (4).

8. Implant médical (1) selon l'une des revendications précédentes, la saillie de positionnement (32) présentant une hauteur de saillie (H) comprise dans une plage de 1 mm à 15 mm, de préférence de 2 mm à 5 mm.

9. Implant médical (1) selon l'une des revendications précédentes, la plaque postérieure (4) présentant des premiers trous de fixation (48) qui sont prévus pour recevoir des premiers moyens de liaison, la plaque crânienne (2) présentant des deuxièmes trous de fixation (50) qui sont prévus pour recevoir des deuxièmes moyens de liaison, et les premiers trous de fixation (48) ou les deuxièmes trous de fixation (50) étant réalisés sous la forme de trous ovales (52), un axe longitudinal (AL) des trous ovales (52) étant de préférence orienté sensiblement le long d'une direction de ceinture pelvienne (RB).

10. Implant médical (1) selon l'une des revendications précédentes, le bras de liaison (6, 8) présentant une rigidité inférieure à celle de la plaque postérieure (4) et de la plaque crânienne (2), de sorte qu'une orientation de la plaque postérieure (4) par rapport à la plaque crânienne (2) peut être ajustée par déformation plastique du bras de liaison (6, 8).

11. Implant médical (1) selon la revendication 1, la plaque crânienne (2) étant de configuration convexe dans un plan transversal et/ou la plaque postérieure (4) étant de configuration convexe dans le plan transversal.

12. Implant médical (1) selon l'une des revendications précédentes, une première portion d'implant (18) destinée à être fixée au premier os pubien (20) et une deuxième portion d'implant (22) destinée à être fixée au deuxième os pubien (24) étant configurées sensiblement symétriques en image miroir l'une par rapport à l'autre, et/ou une longueur (L2) de la plaque postérieure (4) et une longueur (L1) de la plaque crânienne (4) étant sensiblement identiques.

13. Implant médical (1) selon l'une des revendications précédentes, l'au moins un bras de liaison (6, 8) étant disposé dans une direction de ceinture pelvienne (RB) à distance des extrémités (28a, 28b) de la plaque postérieure (4) et/ou des extrémités (26a, 26b) de la plaque crânienne (2).

14. Procédé (100) d'ostéosynthèse d'une disjonction symphysaire sur un corps d'entraînement (64) à des fins d'entraînement au moyen d'un implant médical (1) selon l'une des revendications 1 à 13 précédentes, le corps d'entraînement (64) n'étant pas un corps humain ou animal vivant, le procédé (100) comprenant les étapes suivantes:
- application (S1) d'une plaque crânienne (2) de l'implant médical (1) sur un premier os pubien (20) du corps d'entraînement (64) depuis un côté crânien (44);
- application (S2) d'une plaque postérieure (4) de l'implant médical (1) sur un premier os pubien (20) du corps d'entraînement (64) depuis un côté postérieur (46);
- fixation (S4) de la plaque crânienne (2) au premier os pubien (20);
- fixation (S5) de la plaque postérieure (4) au premier os pubien (20);
- ajustage d'un écart symphysaire (S6) entre le premier os pubien (20) et le deuxième os pubien (24);
- fixation (S7) de la plaque crânienne (2) au deuxième os pubien (24) et fixation (S8) de la plaque postérieure (4) au deuxième os pubien (24) après l'ajustage de l'écart symphysaire (S6).

15. Procédé (100) selon la revendication 14, comprenant en outre l'étape suivante:
- application (S3) d'une saillie de positionnement (32) de l'implant médical (1) sur le premier os pubien (20) depuis un côté (66) orienté vers la symphyse (34) du corps d'entraînement,
l'application (S3) de la saillie de positionnement (32) étant effectuée avant la fixation (S4, S5) de la plaque crânienne (2) et/ou de la plaque postérieure (4) sur le premier os pubien (20).
